# EUROPÄISCHE PATENTSCHRIFT

(11) **EP 1 109 513 B1**
(45) Veröffentlichungstag und Bekanntmachung des Hinweises auf die Patenterteilung: **15.12.2004**
(21) Anmeldenummer: 99934545.7
(22) Anmeldetag: 29.06.1999
(51) Int. Cl.: A61F 2/30, A61N 7/00

(54) **GELENK-ENDOPROTHESE**
JOINT-ENDOPROSTHESIS
ENDOPROTHESE D'ARTICULATION

(30) Priorität: 17.07.1998 DE 19832272
(43) Veröffentlichungstag der Anmeldung: 27.06.2001
(73) Patentinhaber: CBM Cross Border Management Unternehmensberatung GmbH, 86911 Diessen (DE)
(72) Erfinder: HAGENMEYER, Klaus, D-86911 Diessen (DE)
(74) Vertreter: Rupprecht, Kay, Dipl.-Ing.
(86) Internationale Anmeldenummer: PCT/EP1999/004489
(87) Internationale Veröffentlichungsnummer: WO 2000/003663

(56) Entgegenhaltungen:
- US-A- 5 496 256
- US-A- 5 524 624
- US-A- 5 556 372
- US-A- 5 730 705
- US-A- 5 752 924

## Beschreibung

Die Erfindung betrifft eine Gelenk-Endoprothese mit einem Schaft, der in dem Markraum eines Röhrenknochens verankert wird.

Derartige Gelenk-Endoprothesen sind bekannt und dienen beispielsweise dem Ersatz der Schulter-, Arm-, Hüft-, Knie- oder Fußgelenke beim Menschen. All jenen künstlichen Gelenken ist gemeinsam, daß sie wenigstens einen konischen Schaft aufweisen, der beim Einsetzen der Endoprothese zu dessen Fixierung in den Markraum des entsprechenden Röhrenknochens eingeschlagen wird. Meistens weist die Außenmantelfläche des Schaftes Tragrippen auf, die beim Einschlagen zu einer ratschenartigen Verhaftung des Schaftes im Markraum des Röhrenknochens führen. Durch zusätzliches Einbringen von kleinen, weichen Spongiosabröckchen in die Tragrippenbuchten wird die primäre Stabilisierung und das Einwachsen des Knochens nach dem Einsetzen gefördert.

Es hat sich jedoch gezeigt, daß das Einbringen der Spongiosabröckchen nicht immer zu der gewünschten Stabilisierung des Endoprothesenschaftes führt. Das kann zum einen an einer nicht optimalen Paßform des Schaftes in bezug auf den selten ideal geformten Knochen liegen, und zum anderen an dem von Patient zu Patient unterschiedlichen Stoffwechselprozeß mit der damit verbundenen unterschiedlichen Osteogenese. Dadurch, oder auch durch unachtsame Bewegungen beim Tragen der längst eingesetzten Gelenk-Endoprothese, kann es zu Luxationen der Endoprothese mit einer Dislokation von mehreren Zentimetern kommen. In solchen Fällen ist häufig eine erneute, meist in zwei Stufen erfolgende Operation erforderlich.

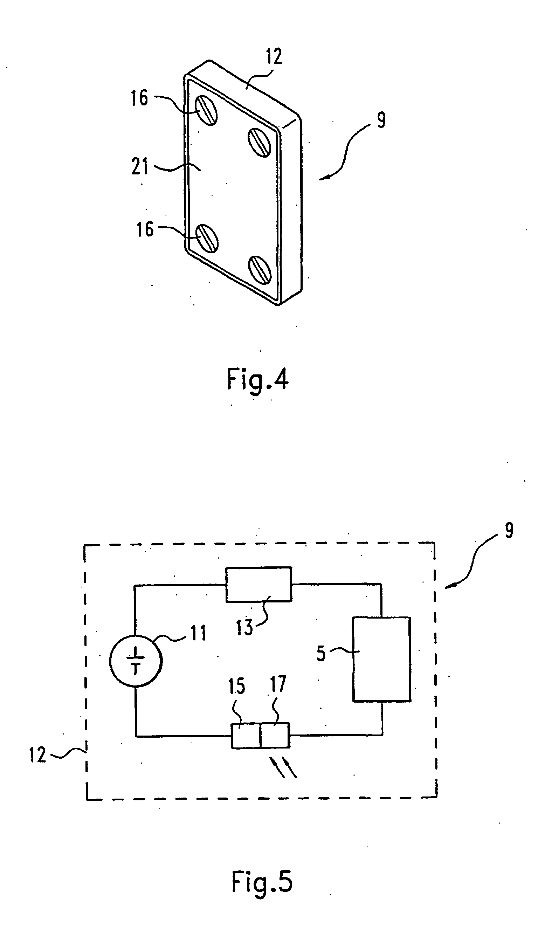

Aus der US-A-5 730 705 ist ein Ultraschallgerät mit einem transportablen Bediengerät und einem Ultraschallkopf bekannt, der auf die Haut des Patienten in der Nähe des mit Ultraschall zu beaufschlagenden Bereichs aufsetzbar ist. Die Ausbreitung der Ultraschallwellen sowie die Ausbildung von Scherwellen entlang des Endoprothesenschaftes sind in den dortigen Fig. 4 und 5 dargestellt. Somit wird mit diesem bekannten Ultraschallgerät der Spalt zwischen der Spongiosa des Röhrenknochens und der Außenmantelfläche des Endoprothesenschaftes mit ultraschall beaufschlagt.

Ein zentrales Problem beim Tragen der Gelenk-Endoprothese ist also die Festigkeit des Sitzes des Endoprothesenschaftes im Knochen. An dieser Problemstellung setzt die vorliegende Erfindung an, als deren Aufgabenstellung es angesehen wurde, eine Gelenk-Endoprothese der vorstehend beschriebenen Art derart weiterzubilden, daß eine bessere Stabilisierung des Schaftes im Knochen erzielbar ist.

Diese Aufgabe wird bei einer Gelenk-Endoprothese der vorstehend beschriebenen Art erfindungsgemäß dadurch gelöst, daß eine Ultraschallquelle an oder in dem Schaft vorgesehen ist, deren Abstrahlcharakteristik derart ausgelegt ist, daß in einen Spalt zwischen der Spongiosa des Röhrenknochens und der Außenmantelfläche des Schaftes Ultraschallwellen gelangen.

Die Erfindung macht sich die bei der Heilung von Knochenfrakturen gewonnene Erkenntnis zunutze, daß sich die zur Heilung einer Fraktur benötigten Osteozyten durch Einleiten von Ultraschallwellen in den Frakturspalt vermehren und dadurch zu einer schnelleren, besseren und festeren Heilung des Frakturspalts führen. Hintergrund dieser Wirkung ist es, daß der Ultraschall die Zellwände der Mesenchymzellen zum Schwingen anregt, woraus eine wünschenswerte Proliferation resultiert. Wesentlich bei der Anwendung von Ultraschall ist, daß er in den Frakturspalt eingeleitet wird, da er dort auf die Spongiosa und das Knochenmark trifft und diese anregt, während er ansonsten von der Kortikalis des Knochens mehr oder weniger reflektiert würde.

Durch die erfindungsgemäße Verbindung des Gelenk-Endoprothesenschafts mit einer Ultraschallquelle und durch deren besondere Abstrahlcharakteristik, die auf den Spalt zwischen der Spongiosa und dem Schaft gerichtet ist, kommt es beim Betrieb der Ultraschallquelle zu der gewünschten Anregung der Zellwände und zur Proliferation der Osteozyten. Dabei kann die Ultraschallquelle außen an dem Schaft angebracht sein, oder aber - was die bevorzugte Ausführungsform sein dürfte - Bestandteil des Schaftes oder aber zumindest in diesen integriert sein. Es können auch mehrere Ultraschallquellen über den Umfang des Gelenk-Endoprothesenschaftes verteilt sein, falls nur dadurch eine gleichmäßige Beaufschlagung des Spalts zwischen Schaft und Knochen erzielbar ist.

Vorteilhafte Weiterbildungen der Erfindung sind in den Unteransprüchen angegeben.

So ist beispielsweise vorgesehen, daß die Ultraschallquelle derart angeordnet ist, daß der Schaft als Schwingungsträger in Form eines Resonanzkörpers dient. Ziel dieser Weiterbildung ist es, eine gleichmäßige Beaufschlagung des Spalts zwischen Schaft und Knochen mit Ultraschall zu erzielen, indem der Schaft den Ultraschall gleichmäßig in den Spalt einleitet.

Vorzugsweise ist jede Ultraschallquelle Teil eines Ultraschallmoduls mit einer eigenen Spannungsquelle, so daß zum Betrieb der Ultraschallquelle keine Spannung von außen zugeführt werden muß, also ein entsprechender Anschluß am Körper des Patienten oder aber ein operativer Eingriff entfällt.

Um eine zu große Wärmeentwicklung durch die Beaufschlagung des Spalts mit dem hochfrequenten Ultraschall (etwa 1,5 MH) zu vermeiden, ist vorgesehen, daß das Ultraschallmodul einen Multivibrator aufweist, mit dem die Ultraschallquelle gepulst, also intermittend betrieben wird. Ein Verhältnis zwischen Betriebsdauer zu Ruhepause von 2:8 hat sich als praktikabel erwiesen.

Ziel der folgenden Weiterbildung ist es, das Ultraschallmodul für einen möglichst langen Zeitraum ohne das Erfordernis eines erneuten operativen Eingriffs betriebsbereit zu halten und von außen per Fernbedienung bedienbar zu machen. Dafür ist vorgesehen, daß das Ultraschallmodul einen Schalter zum Ein/Ausschalten der Ultraschallquelle aufweist, und einen Sensor zum ferngesteuerten Aktivieren des Schalters mittels eines Signalgebers. Dieser Signalgeber kann in bekannter Weise ebenfalls auf Ultraschallbasis oder aber auf Infrarotbasis arbeiten.

Um vor dem Einsetzen der Gelenk-Endoprothese ein möglichst einfaches Einsetzen des Ultraschallmoduls in den Schaft der Gelenk-Endoprothese zu gewährleisten, beispielsweise um vorher noch die Spannungsquelle zu aktivieren, weist das Ultraschallmodul ein Gehäuse auf, das von außen durch eine Abdeckplatte im Schaft zugänglich ist. Diese Abdeckplatte ist durch das Lösen einiger Schrauben abnehmbar.

Trotz einer Minimierung des Stromverbrauchs des Ultraschallmoduls kann es erforderlich sein, die Spannungsquelle von außen aufladbar zu gestalten.

Im folgenden werden zwei bevorzugte Ausführungsbeispiele der Erfindung in Form einer Hüftgelenk-Endoprothese anhand einer Zeichnung näher erläutert.

Es zeigen:
- Fig. 1: einen schematischen ventral-dorsalen Vertikalschnitt durch einen resektierten Femurabschnitt mit eingesetzter Hüftgelenk-Endoprothese;
- Fig. 2: einen schematischen lateral-medialen Vertikalschnitt entsprechend der Fig. 1;

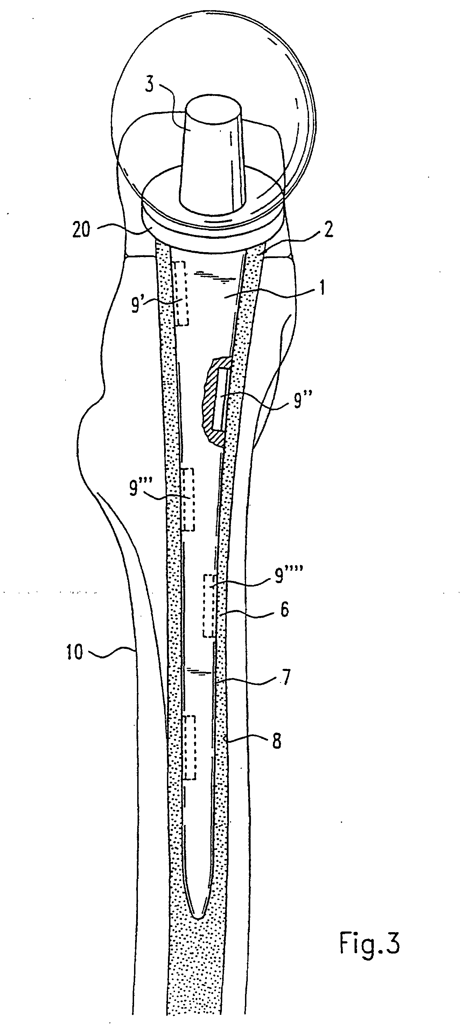

- Fig. 3: eine der Fig. 1 entsprechende Darstellung, jedoch mit mehreren über den Umfang des Schafts verteilten Ultraschallquellen beziehungsweise Ultraschallmodulen;
- Fig. 4: eine schematische Perspektivansicht eines Ultraschallmoduls gemäß dem Detail "X" der Fig. 2; und
- Fig. 5: ein schematisches Schaltbild eines Ultraschallmoduls.

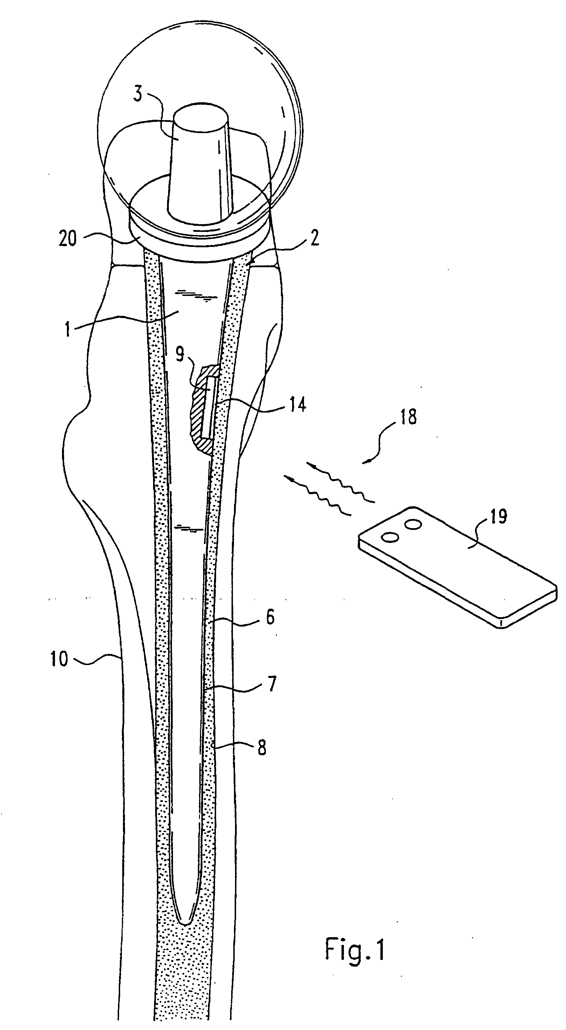

Fig. 1 zeigt einen schematischen ventral-dorsalen Vertikalschnitt durch einen resektierten Femurabschnitt 4, in dessen Markraum 2 ein Schaft 1 einer Hüftgelenk-Endoprothese eingesetzt ist. An dem Schaft 1 setzt nach oben in üblicher Weise eine kegelstumpfförmige Pfanne 3 an, die mit einem Kragen 20 den resektierten Femurstumpf überdeckt. Es ist - zur besseren Erläuterung in übertriebener Größe - ein Spalt 6 zwischen der Außenmantelfläche 7 des Schafts 1 und dem Innenumfang des Markraums 2 dargestellt. Ein solcher Spalt 6 kann beispielsweise beim Einsetzen der Hüftgelenk-Endoprothese durch eine nicht optimale Paßform entstehen, oder aber durch eine Luxation mit der Folge einer Dislokation, hervorgerufen durch eine unachtsame Bewegung oder übermäßige sportliche Betätigung des Trägers der Endoprothese. Der Röhrenknochen 4 des Femurabschnitts weist in bekannter Weise eine relativ harte Außenschicht, die Korikalis 10, auf, und eine relativ weiche, poröse Innenschicht, die Spongiosa 8. Darüber hinaus ist der Markraum 2 und damit der Spalt 6 mit Knochenmark gefüllt.

In dem Endoprothesenschaft 1 ist ein Ultraschallmodul 9 angeordnet, das als wesentliche Bestandteile eine Ultraschallquelle 5, eine Spannungsquelle 11, einen Multivibrator 13, einen Schalter 15 und einen Sensor 17 in einem Gehäuse 12 enthält (vergleiche Fig. 5). Das Gehäuse 12 ist an der Außenmantelfläche 7 des Schafts 1 mit einer ultraschall-durchlässigen Abdeckplatte 14 verschlossen. Die Abstrahlcharakteristik der Ultraschallquelle 5 ist derart ausgelegt, daß beim Betrieb des U1-traschallmoduls Ultraschallwellen in den Spalt 6 zwischen der Spongiosa 8 des Röhrenknochens 4 und der Außenmantelfläche 7 des Schaftes 1 gelangen und dort die Spongiosa 8 und das (nicht dargestellte) Knochenmark zur Proliferation von Osteozyten anregen.

Das Ein- und Ausschalten der Ultraschallquelle 5 erfolgt vorzugsweise mittels einer Fernbedienung in Form eines Signalgebers 19, der zum Aktivieren des Schalters 15 über den Sensor 17 Infrarot- oder Ultraschallstrahlung 18 abgibt.

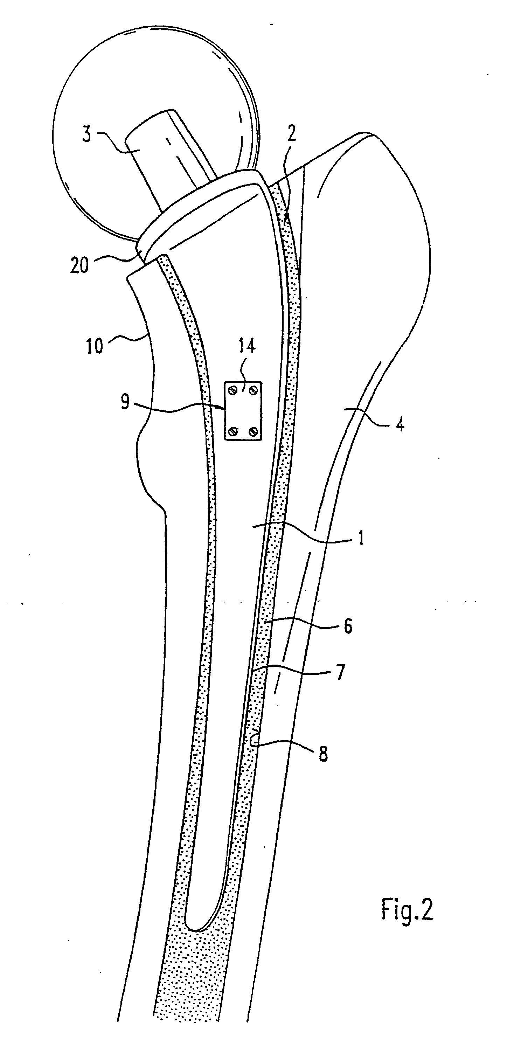

Fig. 2 zeigt einen schematischen lateral-medialen Vertikalschnitt durch den Röhrenknochen 4 gemäß Fig. 1, wodurch der Blick auf die Abdeckplatte 14 des Ultraschallmoduls 9 möglich wird.

Mit dem Ziel, den Spalt 6 um den Schaft 1 herum möglichst gleichmäßig mit Ultraschallenergie zu versorgen ist gemäß einer zweiten Ausführungsform ein Schaft der Gelenk-Endoprothese mit mehreren, über den Umfang des Schafts 1 verteilten Ultraschallquellen 5', 5", 5'''... vorgesehen. Jede Ultraschallquelle ist wiederum Teil eines Ultraschallmoduls 9', 9'', 9"'..., die alle gemeinsam in der vorstehend erläuterten Art ein- oder ausgeschaltet werden können.

Fig. 4 zeigt eine perspektivische Darstellung eines Ultraschallmoduls 9 mit dem Gehäuse 12 und einer Frontplatte 21, die mit Schrauben 16 an dem Gehäuse 12 befestigt ist. Diese Frontplatte 21 kann im eingebauten Zustand des Ultraschallmoduls 9 mit der Abdeckplatte 14 identisch sein.

Fig. 5 zeigt ein schematisches Schaltbild eines Ultraschallmoduls 9. Wie bereits vorstehend beschrieben, weist das Modul eine Spannungsquelle 11, einen Multivibrator 13, eine Ultraschallquelle 5, einen Schalter 15 sowie einen Sensor 17 auf, welche sämtlichst in dem Gehäuse 12 untergebracht sind. Dabei ist die Ultraschallquelle 5 in bezug auf die Abdeckplatte 14 beziehungsweise in bezug auf den Endoprothesenschaft 1 derart angeordnet, daß einen optimale Beschallung des Spalts 6 mit der darin befindlichen Spongiosa 8 und dem Knochenmark sichergestellt ist.

Ein Verfahren zur Anwendung der erfindungsgemäßen Gelenk-Endoprothese wird nun kurz anhand der Fig. 1 erläutert: Unter Ausnutzung der Erkenntnis, daß die Bildung von Osteozyten, welche beispielsweise zum Heilen einer Knochenfraktur benötigt werden, durch die Beaufschlagung der Mesenchymzellen mit Ultraschall gefördert wird, sieht das Verfahren zum Festigen des Sitzes eines Gelenk-Endoprothesenschaftes 1 in dem Markraum 2 eines Röhrenknochens 4 erfindungsgemäß vor, daß der Spalt 6 zwischen der Spongiosa 8 des Röhrenknochens 4 und der Außenmantelfläche 7 des Schaftes 1 mittels einer Ultraschallquelle 5 mit Ultraschall beaufschlagt wird. Zur Vermeidung übermäßiger Wärmeentwicklung im Spalt 6 erfolgt die Beaufschlagung mit Ultraschall gepulst, so daß nach einer Beaufschlagung von ca. 200 µsec Dauer eine Pause von ca. 800 µsec. erfolgt. Eine gängige Ausgangsleistung der Ultraschallquelle 5 liegt bei etwa 30 mW bei einer Schallfrequenz von etwa 1,5 MHz.

## Patentansprüche

1. Gelenk-Endoprothese, mit einem Schaft (1), der in dem Markraum (2) eines Röhrenknochens (4) verankert wird,
**gekennzeichnet durch**
eine Ultraschallquelle (5) an oder in dem Schaft (1), deren Abstrahlcharakteristik derart ausgelegt ist, daß in einen Spalt (6) zwischen der Spongiosa (8) des Röhrenknochens (4) und der Außenmantelfläche (7) des Schaftes (1) Ultraschallwellen gelangen.

2. Gelenk-Endoprothese nach Anspruch 1,
**dadurch gekennzeichnet, daß** die Ultraschallquelle (5) derart angeordnet ist, daß der Schaft (1) als Schwingungsträger dient.

3. Gelenk-Endoprothese nach Anspruch 1,
**gekennzeichnet durch** mehrere Ultraschallquellen (5', 5", 5"'...), die über den Umfang des Schaftes (1) verteilt in oder an dem Schaft (1) angeordnet sind.

4. Gelenk-Endoprothese nach einem der Ansprüche 1-3,
**dadurch gekennzeichnet, daß** jede Ultraschallquelle (5, 5', 5'', 5'''...) Teil eines Ultraschallmoduls (9) mit einer eigenen Spannungsquelle (11) ist.

5. Gelenk-Endoprothese nach Anspruch 4,
**dadurch gekennzeichnet, daß** das Ultraschallmodul (9) einen Multivibrator (13) aufweist, mit dem die Ultraschallquelle (5) gepulst wird.

6. Gelenk-Endoprothese nach Anspruch 4 oder 5,
**dadurch gekennzeichnet, daß** das Ultraschallmodul (9) einen Schalter (15) zum Ein-/Ausschalten der Ultraschallquelle (5) aufweist, und einen Sensor (17) zum ferngesteuerten Aktivieren des Schalters (15) mittels eines Signalgebers (19).

7. Gelenk-Endoprothese nach einem der Ansprüche 4-6,
**dadurch gekennzeichnet, daß** das Ultraschallmodul (9) ein Gehäuse (12) aufweist, das von außen durch eine Abdeckplatte (14) im Schaft (1) zugänglich ist.

8. Gelenk-Endoprothese nach einem der Ansprüche 4-7,
**dadurch gekennzeichnet, daß** die Spannungsquelle (11) von außen aufladbar ist.

## Claims

1. A joint endoprosthesis with a shaft (1) which is anchored in the medullary space (2) of a tubular bone (4),
**characterised by**
an ultrasonic source (5) at the or within the shaft (1), whose irradiation characteristic is designed in such a manner that ultrasonic waves reach a gap (6) between the spongiosa (8) of the tubular bone (4) and the outer surface area (7) of the shaft (1).

2. The joint endoprosthesis according to Claim 1,
**characterised in that**
the ultrasonic source (5) is arranged in such a manner that the shaft (1) serves as a vibration carrier.

3. The joint endoprosthesis according to Claim 1,
**characterised by**
several ultrasonic sources (5', 5", 5'",...) which are arranged about the circumference of the shaft (1) within the or at the shaft (1).

4. The joint endoprosthesis according to one of Claims 1 to 3,
**characterised in that**
each ultrasonic source (5', 5", 5"', ...) is part of an ultrasonic module (9) with an own power source (11).

5. The joint endoprosthesis according to Claim 4,
**characterised in that**
the ultrasonic module (9) comprises a multivibrator (13) by means of which the ultrasonic source (5) is pulsed.

6. The joint endoprosthesis according to Claim 4 or 5,
**characterised in that**
the ultrasonic module (9) comprises a switch (15) for switching on and off the ultrasonic source (5), and a sensor (17) for the remote-controlled activation of the switch (15) by means of a signal generator (19).

7. The joint endoprosthesis according to one of Claims 4 to 6,
**characterised in that**
the ultrasonic module (9) comprises a housing (12) which is accessible from the outside via a cover plate (14) in the shaft (1).

8. The joint endoprosthesis according to one of Claims 4 to 7,
**characterised in that** the power source (11) can be charged from the outside.

## Revendications

1. Endoprothèse d'articulation, comportant un fût (1) qui est ancré dans une cavité médullaire (2) d'un os long (4), **caractérisée par** une source d'ultrasons (5) sur ou dans le fût (1), dont la caractéristique de dissipation est conçue de telle sorte que des ondes ultrasonores parviennent dans une fente (6) entre le tissu spongieux (8) de l'os long (4) et la surface enveloppe extérieure (7) du fût (1).

2. Endoprothèse d'articulation selon la revendication 1, **caractérisée en ce que** la source d'ultrasons (5) est agencée de telle sorte que le fût (1) sert de support d'oscillations.

3. Endoprothèse d'articulation selon la revendication 1, **caractérisée par** plusieurs sources d'ultrasons (5', 5", 5"', ...) qui sont agencées dans ou sur le fût (1) en étant réparties sur la périphérie du fût (1).

4. Endoprothèse d'articulation selon l'une des revendications 1 à 3, **caractérisée en ce que** chaque source d'ultrasons (5, 5', 5", 5"') fait partie d'un module d'ultrasons (9) avec une propre source de tension (11).

5. Endoprothèse d'articulation selon la revendication 4, **caractérisée en ce que** le module d'ultrasons (9) présente un multivibrateur (13) par lequel la source d'ultrasons (5) est pulsée.

6. Endoprothèse d'articulation selon l'une ou l'autre des revendications 4 et 5, **caractérisée en ce que** le module d'ultrasons (9) présente un commutateur (15) pour allumer/éteindre la source d'ultrasons (5), et un capteur (17) pour activer à distance le commutateur (15) au moyen d'un émetteur de signal (19).

7. Endoprothèse d'articulation selon l'une des revendications 4 à 6, **caractérisée en ce que** le module d'ultrasons (9) présente un boîtier (12) qui est accessible depuis l'extérieur à travers une plaque de recouvrement (14) dans le fût (1).

8. Endoprothèse d'articulation selon l'une des revendications 4 à 7, **caractérisée en ce que** la source de tension (11) peut être rechargée depuis l'extérieur.
